# FASCICULE DE BREVET EUROPEEN

(11) **EP 0 976 715 B1**
(45) Date de publication et mention de la délivrance du brevet: **05.02.2003**
(21) Numéro de dépôt: 99401865.3
(22) Date de dépôt: 22.07.1999
(51) Int. Cl.: C07C 67/347, C07C 69/587, C07C 67/303, C10M 105/34

(54) **Procédé pour la codimérisation de diènes de corps gras et d'oléfines catalysée par des complexes au nickel**
Verfahren zur Codimerisierung von Dienfettverbindungen mit Olefinen katalysiert mit Nickelkomplexen
Process for the codimerisation of dienic fatty compounds and olefines catalysed by nickel complexes

(30) Priorité: 28.07.1998 FR 9809671
(43) Date de publication de la demande: 02.02.2000
(73) Titulaire: INSTITUT FRANCAIS DU PETROLE, 92500 Rueil Malmaison (FR)
(72) Inventeur: Hillion, Gérard, 95220 Herblay (FR); Olivier-Bourbigou, Hélène, 92500 Rueil Malmaison (FR); Siepen, Katja, 40699 Erkrath (DE); Commereuc, Dominique, 92190 Meudon (FR); Stern, Robert, 78700 Conflans Sainte Honorine (FR)

(56) Documents cités:
- EP-A- 0 894 785
- WO-A-91/11428
- US-A- 5 434 282

## Description

L'invention a pour objet un nouveau procédé d'obtention de composés chimiques issus de corps gras polyinsaturés, composés caractérisés par la présence le long de la chaîne linéaire hydrocarbonée d'une ou de plusieurs ramifications de deux atomes de carbone au moins.

Ces composés sont obtenus par addition d'oléfines sur des corps gras polyinsaturés en présence d'un système catalytique au nickel.

Ces co-dimères insaturés peuvent être hydrogénés et l'on obtient alors des corps gras saturés caractérisés par un point de fusion généralement inférieur à -20 C, une thermostabilité importante et des propriétés tensioactives recherchées.

La présence de ramifications dans les composés à base de corps gras, surtout lorsque ces ramifications se situent vers le milieu des chaînes linéaires comportant de 14 à 18 atomes de carbone, se traduit par un certain nombre de propriétés remarquables, comme par exemple :
- l'abaissement très important des points de fusion, des points d'écoulement et des points de trouble et une augmentation sensible de la viscosité des corps gras ramifiés par rapport aux mêmes composés linéaires non ramifiés ; cette propriété est par exemple utilisée dans les lubrifiants, les graisses ou les plastifiants, où l'on emploie des esters de corps gras, des sels ou des esters d'alcools ramifiés dont l'acide peut être organique ou minéral ;
- la réduction de la tension superficielle et de la tension interfaciale, ces caractéristiques étant toujours recherchées dans le domaine des tensioactifs et des émulsifiants ; cette réduction permet d'obtenir des CMC (Concentration Micellaire Critique) inférieures ;
- l'inhibition de la cristallisation des savons ramifiés, en mélange ou non avec des savons classiques, ce qui permet d'obtenir des savons transparents ;
- une augmentation de l'hydrophilie, ce qui rend les composés ramifiés plus solubles ou plus mouillables ; une utilisation possible serait d'utiliser des sels quaternaires d'acides gras ramifiés dans les adoucissants où l'adoucissement va de pair avec une certaine mouillabilité ;
- une modification de la surface de la molécule, surface caractérisée par des vides qui sont provoqués par la présence de ramifications ; l'application en cosmétique de cette propriété permet d'envisager des formules de crèmes pour la peau qui laissent passer la vapeur d'eau, par exemple des bases constituées d'esters d'acides ramifiés ou même d'esters où l'acide et l'alcool sont tous deux ramifiés ;
- la solubilité accrue des sels de métaux lourds avec des acides ramifiés, ce qui les rend solubles soit dans l'eau soit dans certains solvants organiques ; les applications sont multiples comme agents siccatifs dans les peintures, comme pigments, dans l'extraction des métaux ou l'anticorrosion, où l'on peut utiliser des sels de calcium, d'alcanolamines ou même d'amines comme agents actifs. De même, les sels d'acides ramifiés offrent une compatibilité plus grande de certaines charges minérales avec des polymères, ce qui permet d'augmenter le taux des charges dans les plastiques ;
- l'effet bactéricide ou bactériostat plus ou moins marqué selon la nature des bactéries et le nombre ou la grandeur des ramifications permet de protéger des crèmes contre l'attaque bactérienne ou de remplacer les sels quaternaires dans les formulations basiques ou non ; une autre utilisation existe comme inhibiteur d'évaporation de l'eau, où par exemple des composés comme un alcool ramifié ou un monoglycéride d'acide ramifié permettent de retarder la biodégradabilité donc d'économiser l'inhibiteur.

La réaction des oléfines avec le butadiene ou d'autres diènes est connue depuis longtemps et a fait l'objet de plusieurs revues. La codimérisation du butadiène avec l'éthylène conduit à l'hexadiène-1,4, celle de l'éthylène avec l'isoprène au méthyl-3 hexadiène et enfin, par codimérisation de l'éthylène avec le pipérylène, on obtient le vinyl-2-pentène. De nombreux catalyseurs sont utilisés pour réaliser ces réactions. On peut citer par exemple les systèmes au rhodium, au ruthénium, au palladium, au cobalt, au fer ou au nickel. Les systèmes à base de titane ont été décrits (Connel, Laurence G.-Ann. N.Y. Acad. Sci. (73), 214, 143-9) pour catalyser la formation du vinylcyclobutane à partir d'éthylène et de butadiène.

Par contre, l'addition d'oléfine sur des diènes fonctionnels n'a été que très peu décrite. Le brevet US-A-3 742 080 signale la possibilité d'additionner de l'éthylène à des diènes dont l'une ou les deux extrémités des chaînes hydrocarbonées sont substituées par des groupes halogènes ou alkoxy.

Il est également connu qu'une oléfine peut réagir sur un composé diénique ou triénique conjugué selon une réaction de type Diels-Alder. Il est décrit (R. E. Beal et Coll. JAOCS 52, 400 (1975)) par exemple l'addition d'éthylène sur des corps gras polyinsaturés par simple chauffage à une température de 290 °C. Ainsi, à partir de linoléate de méthyle et d'éthylène, on obtient un composé possédant dans sa chaîne hydrocarbonée un cycle insaturé à 6 atomes de carbone. Après hydrogénation, ces composés possèdent des propriétés intéressantes. Toutefois, leur point de fusion, supérieur à 10 °C, est encore trop élevé pour permettre leur utilisation comme lubrifiants.

On connaît une autre méthode d'obtention de composés ramifiés de corps gras. Elle consiste à faire réagir, selon une réaction de type Wittig, une cétone comme par exemple l'ester méthylique de l'acide 12- oxo octadécanoïque avec un ylure, par exemple l'intermédiaire P(⌀)₃=CHCH₃, où ⌀ représente un radical phényle. On obtient alors le composé CH₃(CH₂)₅C(=CHCH₃)(CH₂)₁₀COOCH₃, qui peut être hydrogéné en éthyl-12-octadécanoate de méthyle. (D.G. Chasin et Coll, Chem. Phys. Lipids (71) 6, 8-30).

On a signalé également dans la nature la présence de composés saturés ramifiés de corps gras tirés des bacilles de Koch par exemple, ou, avec une autre longueur de chaîne hydrocarbonée, dans le suif de mouton.

On sait enfin que les produits que l'on appelle «isostéariques» contiennent des traces de composés portant des ramification de type éthyle ou vinyle.

Récemment, les demandes de brevets internationales WO-A-91/11428, 91/11427, 91/11426 et 91/11425 ont décrit l'obtention de composés de corps gras ramifiés par un procédé catalytique. L'addition de l'oléfine, telle que l'éthylène ou le propylène, sur le corps gras polyinsaturé, un ester de l'acide linoléique par exemple, est catalysée par un système à base de rhodium, d'iridium, de palladium ou de ruthénium. Les systèmes au rhodium, qui sont les seuls à avoir été décrits de façon explicite, restent cependant peu actifs.

Les brevets US-A-5 476 956 et 5 434 282 décrivent l'utilisation d'un système catalytique au rhodium très spécifique, qui permet d'accélérer l'addition de l'oléfine sur les diènes de corps gras, notamment les diènes conjugués, d'un facteur 50 à 100. Toutefois, ce procédé reste encore difficilement applicable à une grande échelle en raison de la consommation trop élevée de rhodium.

La demande de brevet français publiée FR-A-2 766 482, au nom du même déposant, décrit un procédé utilisant un système catalytique au cobalt, qui consiste à faire réagir des oléfines simples, par exemple l'éthylène, sur des esters polyinsaturés, par exemple le linoléate de méthyle conjugué ou non, pour obtenir des esters ramifiés. Les composés ramifiés obtenus peuvent être hydrogénés et être utilisés entre autres comme bases de lubrifiants. Cette demande de brevet décrit un procédé d'obtention d'un codimère dans lequel des co-catalyseurs peuvent éventuellement être introduits, comme par exemple des métaux de transition du type fer, nickel, cuivre, rhodium ou palladium. Ces catalyseurs permettent de catalyser la conjugaison, si l'on part d'un ester polyénique non conjugué, et donc d'accélérer la réaction.

On a maintenant trouvé de façon surprenante qu'en utilisant un simple composé de nickel avec un réducteur acide ou un composé réduit du nickel avec un acide de Bronsted ou de Lewis, on pouvait, en présence d'un ligand, obtenir la réaction principale d'addition d'éthylène ou d'une autre oléfine sur l'ester, conjugué ou non, et ceci dans des conditions particulièrement douces, puisque l'on peut obtenir une conversion totale des esters conjugués à moins de 0,1 MPa de pression d'éthylène et entre 30 et 80 °C. De plus, grâce à la réactivité accrue de ces catalyseurs au nickel, on peut obtenir l'addition de 2 ou 3 radicaux éthylènes sur le substrat diénique.

Le nickel étant fortement conjugant, on peut en une étape également obtenir un produit d'addition en partant d'un ester polyénique non conjugué mais conjuguable.

L'objet de la présente invention est donc un nouveau procédé de codimérisation d'un composé monooléfinique avec un corps gras polyinsaturé en présence d'un système catalytique comprenant un composé de nickel. Grâce à ce type de catalyseur, on peut envisager de réduire fortement le coût de cette réaction, obtenir une sélectivité améliorée par rapport aux produits obtenus avec les complexes au rhodium et une réactivité accrue par rapport aux complexes au cobalt et par conséquent, d'une part, travailler sans problème à pression atmosphérique et, d'autre part, obtenir facilement des composés plus lourds avec l'addition de 2 ou 3 radicaux éthylènes sur les composés polyéniques.

Le composé dit «corps gras polyinsaturé ou polyénique» mis en jeu dans la réaction sur laquelle est basée le procédé de l'invention est en général un composé comprenant d'une part au moins deux liaisons éthyléniques, ces liaisons pouvant être conjuguées ou conjugables deux à deux et d'autre part une fonction carboxylique comme celle présente dans les acides gras ayant de 18 à 26 atomes de carbone. Les huiles de tournesol, de carthame, de poisson, de lin, de soja, d'oïticica, de coton, de colza, de bois de chine, de noix, de maïs, de linola, de pépins de raisin et généralement toutes les huiles ou leurs esters dérivés comprenant des composés polyinsaturés sont des matières premières envisageables.

Les acides gras considérés qu'ils soient diéniques, triéniques ou polyéniques sont utilisés sous la forme de leurs esters, que l'on forme soit à partir d'acides gras ou d'huiles par réaction avec des alcools monofonctionnels, tels que par exemple le méthanol, l'éthanol, le 2-éthylhexanol ou l'isopropanol ; des alcools difonctionnels, tels que par exemple le néopentylglycol ; trifonctionnels, tels que par exemple le triméthylolpropane; et polyfonctionnels, tels que par exemple le sorbitol, les polyglycérols, le pentaérythritol et les sucres. Les huiles elles-mêmes, en tant qu'esters de glycérol, sont des substrats possibles.

Ces esters peuvent, au départ, être conjugués, partiellement ou totalement, ou non conjugués. Autrement dit, ils peuvent contenir au moins deux doubles liaisons éthyléniques séparées ou non entre elles par un groupe méthylénique. Parmi les procédés de conjugaison des doubles liaisons les plus connus, on peut citer ceux qui utilisent les alcoolates alcalins en présence ou non d'un solvant. On peut dans ce cas obtenir jusqu'à 99 % de corps gras conjugué par rapport au corps gras polyinsaturé présent initialement dans l'huile.

On connaît d'autres systèmes catalytiques conjugants, mettant en jeu des complexes de ruthénium ou le fer carbonyle. Le système au nickel est lui-même conjugant et peut être utilisé pendant ou avant la réaction avec l'éthylène.

Le composé monooléfinique mis en jeu dans la réaction peut consister en toute oléfine réactive choisie parmi les monooléfines ordinaires (hydrocarbures monoléfiniques) telles que par exemple l'éthylène, le propylène ou le butène-1 et de préférence l'éthylène.

Le procédé de préparation de corps gras ramifiés est caractérisé en ce que l'on additionne au moins un composé monooléfinique sur un corps gras comportant au moins deux liaisons éthyléniques conjuguées ou non conjuguées, en présence d'un système catalytique comprenant au moins un composé du nickel, au moins un composé réducteur et/ou au moins un acide de Bronsted ou de Lewis et au moins un ligand.

Le composé du nickel est un sel inorganique ou organique bivalent et exceptionnellement monovalent ou zérovalent du nickel. Citons parmi les composés bivalents du nickel, les halogénures, les thiocyanates, les sulfates, les nitrates, les alcoolates, les carbonates, les carboxylates, les bétadicétonates ou les esters d'acides bétacétocarboxyliques ; des exemples particuliers de sels de nickel utilisables sont le chlorure de nickel (II), le bisacétylacétonatonickel (II), l'acétate de nickel (II). Parmi les composés zérovalents, on peut citer le nickel bis-cyclooctadiène [Ni(COD)₂], mais aussi le nickel de Raney.

Comme composé réducteur, on utilise les composés organoaluminiques de formule générale RₓAlX₃₋ₓ ou R₄Al₂SO₄, où R est l'hydrogène ou un groupe alkyle, où x = 1, 2 ou 3, et ou X est un halogène ; les composés organomagnésiens de formule RMgX, avec R = alkyle ou aryle et X = halogène ; et les aluminoxanes. De préférence, on utilisera des composés acides assez forts comme le dichloro éthyl aluminium, le dichloro isobutyl aluminium, soit en général des réducteurs du type AIX₂R avec R = alkyle ; mais ces composés peuvent aussi se former in situ à partir de chlorure de nickel et de dialkylchloroaluminium, ou en présence de chlorure d'aluminium et d'alkylaluminium. Si le composé au nickel est zérovalent, on peut ajouter un acide de Lewis comme HPF₆, HAlCl₄, HBF₄ ou HCI.

Le ligand est choisi :
- parmi les composés du phosphore de formule PRₘX₃₋ₘ, avec m = 2 ou 3 ; R = aryle ou alkyle ; X = halogène ; les phosphites de formule P(OR)₃, avec R = aryle ou alkyle; les oxydes de phosphines de formule POR₃ ; et les diphosphines de formule R₂P-(CH₂)ₙ-PR₂, avec R = aryle ou alkyle et n = 0-4 ;
- parmi les composés analogues de l'arsenic ou de l'antimoine et
- parmi les ligands azotés, comme par exemple les dérivés amidiques, les imines ou diimines (fabriqués par exemple par réaction du glyoxal avec un dérivé de l'aniline substitué sur le noyau aromatique) et enfin les dérivés pyridiniques, tels que par exemple le dipyridyle.

On peut éventuellement mettre en jeu un composé organique qui joue le rôle de solvant ; comme solvants, on peut utiliser les hydrocarbures aliphatiques ou aromatiques, les éthers, les esters, les hydrocarbures halogénés. La réaction peut aussi être réalisée et de préférence en l'absence de solvant. C'est alors l'ester dont une partie ne réagit pas avec l'oléfine qui joue le rôle de solvant ; c'est le cas en particulier avec les esters saturés ou monooléfiniques.

Le rapport molaire entre le ligand et le composé du nickel est compris de préférence entre 0,5/1 et 30/1, en particulier entre 1/1 et 4/1.

Si le ligand coordonne pour un site de coordination, on a intérêt à l'utiliser avec un rapport molaire ligand/nickel compris entre 1/1 et 4/1. Si le ligand est bicoordinant, on l'utilise avec un rapport molaire compris entre 0,5/1 et 2/1.

Le rapport molaire entre le réducteur et le composé du nickel est généralement compris entre 1/1 et 30/1 et de préférence entre 4/1 et 15/1. S'il s'agit d'un acide de Lewis ajouté au nickel déjà réduit, le rapport réducteur sur le nickel peut être dans ce cas de 1/1 à 3/1.

Généralement, on dissout le complexe de nickel comprenant le ligand dans l'ester diénique et, sous atmosphère d'éthylène, on introduit le réducteur. Si l'on veut travailler sous pression d'éthylène, on suit le même mode opératoire en n'introduisant la pression d'éthylène dans l'appareillage qu'après l'ajout du réducteur.

La composition catalytique est ajoutée au système en quantité catalytique. Cette quantité s'exprime comme étant de 10⁻³ à 10⁻¹ mole de nickel par mole de corps gras insaturé, conjugué ou non, et de préférence de 10⁻² à 10⁻³ mole de nickel par mole de diène. La température de réaction est en général de 30 à 120 °C et de préférence de 50 à 80 °C. La pression d'éthylène ou de propylène est de 0,1 à 30 MPa, de préférence de 0,1 à 0,5 MPa. Les temps dépendent de la concentration et de la nature du catalyseur. Ils peuvent être courts, par exemple de quelques minutes à quelques heures. Il est préférable de couper l'arrivée d'éthylène lorsque l'on a consommé le diène afin d'éviter la dimérisation de l'éthylène.

On peut opérer selon un procédé continu ou discontinu. L'introduction du catalyseur et des esters dans le réacteur peut se réaliser en présence d'éthylène à basse température ou à plus haute température directement dans le réacteur.

On peut opérer avec un catalyseur insoluble supporté, par exemple en fixant le nickel sur un support phosphinique hétérogène polymérique, mais aussi en utilisant un nickel de Raney avec des ligands.

Les corps gras ramifiés obtenus peuvent être hydrogénés pour obtenir des produits plus stables. L'hydrogénation des composés oléfiniques est connue depuis près de 30 ans. Elle a été réalisée notamment en 1971 par Chasin, cité plus haut. Les catalyseurs utilisables sont ceux connus pour hydrogéner des oléfines, à savoir le nickel de Raney, le palladium sur charbon, le nickel supporté, généralement après élimination du catalyseur de codimérisation par lavage à l'eau. On peut parfois utiliser le catalyseur de codimérisation comme catalyseur d'hydrogénation. Après hydrogénation, on élimine les composés saturés non ramifiés par cristallisation ou par distillation. On peut aussi distiller avant hydrogénation pour concentrer les produits ramifiés. Cette distillation se fera d'autant plus facilement que l'on aura fixé deux molécules d'éthylène sur le substrat, ce qui permettra de séparer facilement un ester à 22 atomes de carbone d'un ester à 18 atomes de carbone sur la chaîne lipidique. Ces esters à 18 et accessoirement à 16 atomes de carbone qui font partie du substrat de départ et qui ne peuvent réagir sont des esters saturés ou monooléfiniques, comme par exemple les esters de l'acide palmitique, stéarique ou oléique. C'est souvent le cas, si l'on part d'une huile végétale polyoléfinique comme par exemple de l'huile de tournesol, transestérifiée ou non.

Les esters ramifiés peuvent servir de bases de lubrifiants ou d'émulsifiants, ou subir d'autres traitements comme la transestérification avec des alcools plus lourds lorsqu'on a affaire, au départ, à des esters méthyliques.

Enfin, ils peuvent réagir comme les esters habituels en étant soumis à l'hydrogénolyse ou la transformation en amines ou amides.

Les exemples suivants illustrent l'invention ; ils ne sont pas limitatifs.

### EXEMPLE 1 :

Les réactifs sont les suivants:
a) 5,5 g d'un complexe NiCl₂(PBu₃)₂, fabriqué par réaction du chlorure de nickel hydraté et de tributylphosphine, selon une procédure connue. Le poids moléculaire étant de 534, la quantité de 5,5 g correspond à 10,3 mmoles de nickel, soit de l'ordre de 650 ppm (parties par million) par rapport au poids d'ester méthylique engagé.
b) 1000 ml d'ester méthylique d'huile de tournesol préalablement conjugué (EMTC) ; ce volume correspond à 870 g d'ester qui contient de l'ordre de 65 % de linoléate de méthyle conjugué, présent sous la forme 9 cis-11 trans et 10 trans-12 cis.
   Cet ester peut être fabriqué à partir d'un ester méthylique d'huile de tournesol par action d'un alcoolate de potassium à 120-130 °C. Le produit de réaction est lavé, séché et distillé. On obtient un ester dont l'indice d'acide (I.A.) est inférieur à 0,6, ce qui correspond à 1 mmole en acidité oléique/100 g.
   Si l'on ne prend pas les précautions nécessaires pour réduire l'acidité de cet ester conjugué, il y a une consommation plus grande de réducteur.
c) 14 ml ou 96,6 mmoles (d=1,26 à 30 °C) d'éthylaluminium dichlorure (EADC). Le rapport entre le réducteur et le complexe de nickel est de 9 : 1 en mole. L'utilisation de ce réactif exige les précautions d'usage pour le prélèvement, par l'intermédiaire d'une seringue parfaitement sèche.
d) L'éthylène est disponible sous pression dans un ballast de volume connu. Pour suivre avec précision la consommation, il est nécessaire de chauffer le ballast au-dessus du point critique du gaz.

### Partie expérimentale

Dans un réacteur de 3 litres, en verre et à double enveloppe, chauffé par un fluide thermique, on introduit, après séchage et inertage de l'enceinte, la totalité de l'ester conjugué et le complexe au nickel, que l'on agite par l'intermédiaire d'une turbine jusqu'à parfaite dissolution. On obtient alors une solution rouge. On chauffe ce mélange jusqu'à 80 °C et on sature la solution en éthylène par un bullage contrôlé. L'appareillage étant muni d'un septum, on introduit à l'aide d'une seringue en une seule fois la quantité de réducteur (EADC).

Tout au long de la réaction, on maintient l'agitation ainsi que le bullage d'éthylène. Dès qu'il n'y a plus de consommation d'éthylène, on neutralise l'activité catalytique par addition d'eau et de méthanol.

Des prises d'échantillons sont effectuées toutes les heures, où, pour un volume soutiré de 2 ml de solution, on traite l'échantillon avec 2 ml de méthanol, 1 ml d'eau et 3 ml de n-heptane, afin de détruire l'excès de réducteur. La phase organique supérieure est analysée en chromatographie en phase gazeuse.

### Analyse par CPG (chromatographie par perméation de gel)

On obtient le maximum de renseignements en utilisant deux colonnes adéquates.

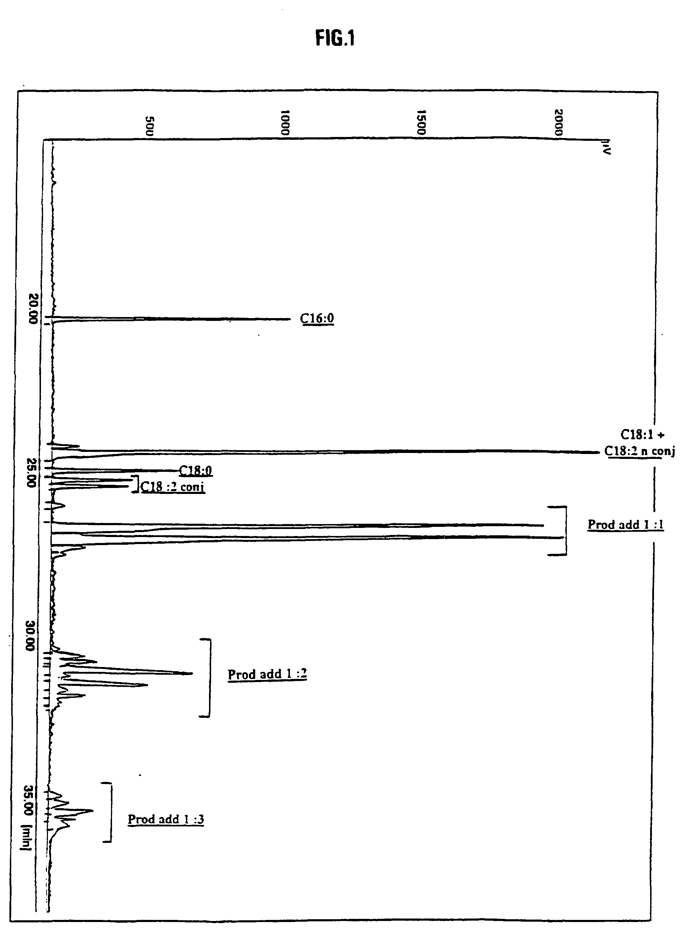

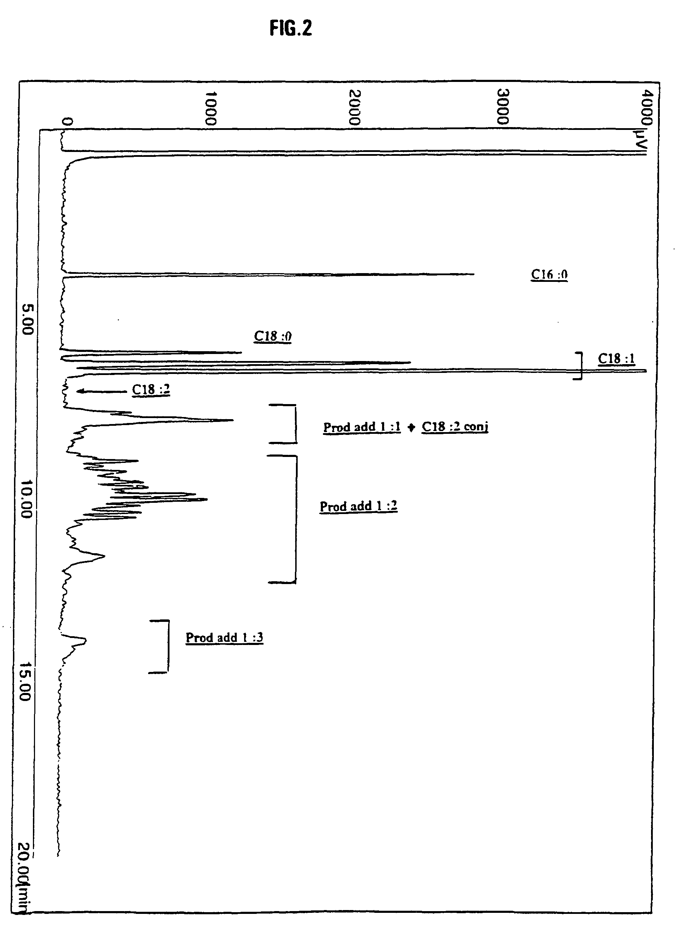

La première est une colonne capillaire de 50 m de type PONA, qui permet d'analyser les composés obtenus par l'addition d'un, deux ou trois éthylènes ajoutés à l'ester diénique. On sépare facilement les familles de produits à 18, 20, 22 et 24 atomes de carbone, mais par contre on n'arrive pas à obtenir la séparation entre le C18 : 1 (oléique) et le C18 : 2 non conjugué (linoléique) (Planche 1 / 2)

La deuxième est une colonne capillaire de 50 m appelée BPX70 (phase très polaire), qui sépare tous les isomères des esters en C18, mais au détriment des composés d'addition, qui sont très mal différenciés. Un exemple de chromatogramme est donné en annexe (Planche 2/2).

### Purification du produit de réaction et méthodes utilisées pour isoler les produits d'addition.

En fin de réaction, la solution d'ester est noire. Cette solution est traitée au méthanol, puis à l'eau, afin de détruire la totalité du réducteur (EADC : éthyl aluminium dichlorure). Au cours de ce traitement, il se produit un important dégagement gazeux et la couleur de la solution passe du brun au jaune avec la formation d'un important précipité blanc d'hydroxyde d'aluminium. La séparation de la phase ester ainsi débarrassée du catalyseur est facilitée par l'ajout de n-heptane.

Après évaporation du solvant, la phase ester est distillée sous pression réduite. On élimine une fraction de tête, qui représente environ 5 % en volume du produit à distiller. Dans cette fraction, on récupère une partie du ligand (tributyl phosphine) et un mélange d'esters méthyliques comportant des chaînes en C16 et en C18. La deuxième fraction qui est récupérée a été distillée sous un vide dynamique de l'ordre de 2 mm de mercure et à une température comprise entre 170 et 210 °C.

Le résidu de distillation représente environ 5% en volume du produit de départ.

On introduit le produit distillé dans un autoclave de 1 litre afin d'hydrogéner la totalité des doubles liaisons à l'aide d'un catalyseur au nickel (catalyseur généralement utilisé pour l'hydrogénation des huiles végétales). L'hydrogénation s'effectue à 200 - 210 °C sous 3 MPa d'hydrogène. Après 5 heures de réaction, il n'y a plus de consommation d'hydrogène.

Pour isoler les esters ramifiés, on élimine par cristallisation fractionnée dans l'acétone les esters saturés qui sont principalement du palmitate et du stéarate de méthyle. C'est le palmitate de méthyle qui est l'impureté la plus difficile à éliminer. On obtient ainsi un produit liquide qui contient environ 95 % de produits d'addition. Le rendement de la cristallisation est très bon. Après un lavage à l'acétone froide, le produit solide cristallisé contient moins de 0,5 % de produits d'addition.

| Esters méthyliques d'acides gras | Avant hydrogénation | Après hydrogénation |
|---|---|---|
| C16 : 0 (ac. palmitique) | 6,10 | 5,90 |
| C18 : 0 (ac. stéarique) | 3,90 | 33,10 |
| C18 : 1 (ac. oléique) | 28,80 | 0 |
| C18 : 2 (ac. linoléique) | 0 | 0 |
| Produit add 1 : 1 | 19,90 | 17,40 |
| Produit add 1 : 2 | 37,70 | 39,40 |
| Produit add 1 : 3 | 3,60 | 4,20 |

### EXEMPLE 2 :

Les réactifs sont les suivants:

| | |
|---|---|
| NiCl₂(PMe₃)₂ | 0,35 mmol |
| EADC | 10,5 mmol |
| Ester méthylique (65 % de C18 :2) | 58,0 mmol (en C18 :2 conj) ou 30 ml d'ester |

Les conditions opératoires sont les suivantes

| | | |
|---|---|---|
| T = 80 °C ; | Pression d'éthylène = 3 MPa ; | temps = 3 heures. |

Dans la solution d'ester à 80 °C qui contient le complexe au nickel, on introduit sous une faible pression d'éthylène le réducteur. Ensuite, on met sous une pression de 3 MPa d'éthylène l'appareillage tout en agitant le mélange par l'intermédiaire d'un barreau magnétique.

Après 3 heures de réaction, les résultats exprimés en % poids sont les suivants :

| | |
|---|---|
| C16: 0 = 6,60 | prod 1 : 1* = 4,30 |
| C18 : 0 = 4,40 | prod 1 : 2* = 22,3 |
| C18 : 1 = 21,0 | prod 1 : 3* = 42,0 |

| | |
|---|---|
| * produits d'addition de 1*, 2* ou 3* éthylènes | |

### EXEMPLE 3 :

On utilise à pression atmosphérique d'éthylène un complexe de type: 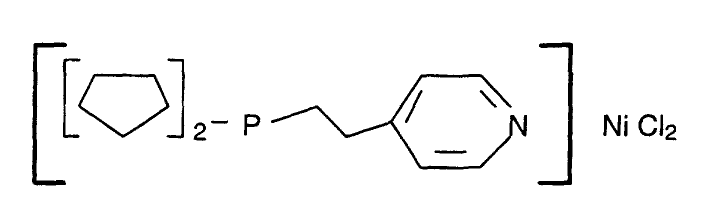 avec un réducteur de type EADC . Après 3 heures de réaction, il se forme de l'ordre de 12 % poids de produit d'addition, soit 23 % de conversion par rapport au linoléate conjugué présent dans le mélange d'esters méthyliques de l'huile de tournesol.

### EXEMPLE 4 :

D'autres ligands ont été utilisés comme le triméthylphosphite et le triphényl phosphite.

En utilisant les quantités de réactifs ci-après ;
Ni(acétylacétonate)₂ = 0,05 mmol, ligand = 0,1 mmol, ester conjugué = 5 ml, réducteur (EADC) = 0,3 ml, on obtient, après 3 heures de catalyse à 80 °C sous 3 MPa de pression d'éthylène, les résultats suivants
- avec P(OCH₃)₃ on arrive à 35 % de conversion en produit d'addition 1 : 1
- et avec P(O-phényl)₃ de l'ordre de 13% de conversion par rapport au linoléate de méthyle conjugué présent dans l'ester.

### EXEMPLE 5 :

On peut utiliser à la place de l'éthylaluminium dichlorure, l'isobutylaluminium dichlorure.

Les réactifs sont les suivants:
- 0,5 mmol de NiCl₂(PBu₃)₂ ou de NiCl₂[P(cyclohexyl)₃]₂ ou de NiCl₂[P(isopropyl)₃]₂
- 5 mmol d'isobutyl aluminium dichlorure
- 50 ml d'ester méthylique de tournesol contenant 65 % de linoléate de méthyle conjugué.

Les conditions opératoires sont les suivantes :

| | | |
|---|---|---|
| température = 80 °C ; | P = 3 Mpa ; | temps = 3 h. |

Les résultats obtenus sont les suivants:

| Complexes | temps | C16 :0 | C18 :0 | C18 :1+2 | Prod 1:1 | Prod 1:2 | Prod 1:3 |
|---|---|---|---|---|---|---|---|
| NiCl₂(P-Bu₃)₂ | 4 h | 7,0 | 5,3 | 24,5 | 13,5 | 16,9 | 32,8 |
| NiCl₂(P-Cycl₃)₂ | 4 h | 6,9 | 4,8 | 71,5 | 16,8 | ― | ― |
| NiCl₂(P-lsPr₃)₂ | 5 h | 6,6 | 4,7 | 71,7 | 17,0 | ― | ― |

### EXEMPLE 6 :

On peut aussi utiliser un réducteur moins acide comme le diéthylaluminium chlorure (DEAC). A une température de 80 °C, sous une pression de 3 MPa et avec 60 ml d'ester méthylique de tournesol conjugué, on obtient, avec le complexe NiCl₂(PBu₃)₂ et pour une stoechiométrie Ni/DEAC = 1 : 15, une conversion en produit d'addition 1 :1 de l'ordre de 23 %.

### EXEMPLE 7 : (comparatif)

On peut également engager un complexe déjà réduit, puis ajouter un réducteur.

Lorsqu'il n'y a pas addition d'un ligand, il ne se forme pas de produits d'addition.
Avec les conditions suivantes :
Température = 80 °C, P = 3 MPa
et en utilisant les réactifs suivants :
- Ni(COD)₂ 0,35 mmol (COD = cyclooctadiène)
- EADC 10,5 mmol Rapport molaire = 1/30
- Ester conjugué 30 ml
on n'obtient aucune conversion après 3 heures de réaction.

### EXEMPLE 8 :

Les mêmes conditions que dans l'exemple 7 sont reproduites, mais en introduisant en plus une mole de triphényl phosphine par rapport au nickel. On obtient, dans ce cas, un produit de réaction dont la composition, exprimée en % poids, est la suivante:

| | |
|---|---|
| C16 : 0 | 6,0 |
| C18 : 0 | 4,2 |
| C18 : 1 | 20,3 |
| C18 : 2 | 1 6 |
| C18 : 2 conj | 24,4 |
| prod add 1 : 1 | 32,8 |
| prod add 1 : 2 | 10,5 |
| prod add 1 : 3 | traces |

Le rendement exprimé en % poids, obtenu par rapport au linoléate de méthyle conjugué, est de l'ordre de 63 %.

### EXEMPLE 9 :

On peut utiliser également un ester éthylique conjugué qui est facilement fabriqué à partir d'un ester éthylique d'huile de tournesol ou encore par estérification d'acides gras conjugués.

Les réactifs sont les suivants:
- NiCl₂(PBu₃)₂ 10 g ou 0,019 mol
- EADC 24 ml ou 0,232 mol (Ni/réd = 1 :12)
- Ester éthylique conjugué 1500 ml (l'acidité de cet ester est inférieure à 1 mmol/100 g).

Les conditions de l'essai sont les suivantes :

| | | |
|---|---|---|
| Température = 80 °C ; | P = 0,1 MPa ; | Temps de catalyse = 8 h. |

En fonction du temps de catalyse, on obtient les résultats suivants.

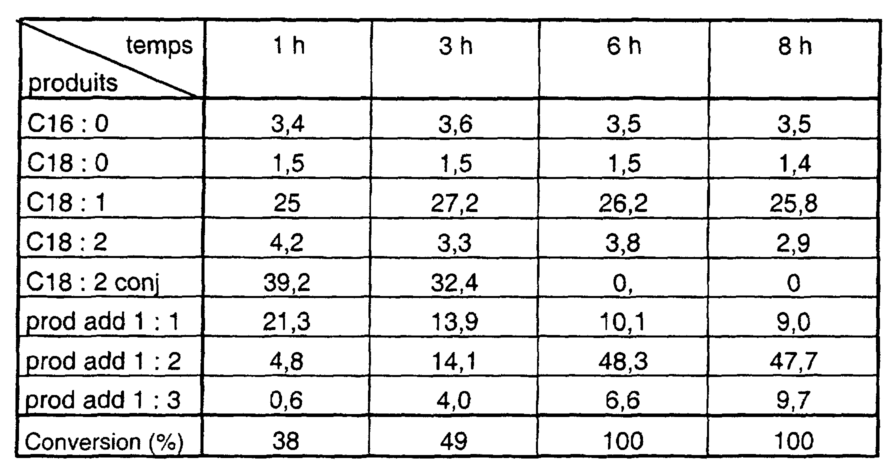

On constate qu'il se forme des esters plus lourds avec addition de 2 moles d'éthylène avant même que l'ester conjugué soit épuisé.

Par conséquent, on peut imaginer que le catalyseur est très conjugant ou isomérisant, ce qui d'ailleurs est visible sur le chromatogramme en CPG avec l'apparition de nouveau pics de C18 : 1.

### EXEMPLE 10 :

On n'est pas obligé de partir d'un ester conjugué.
Avec le complexe de nickel ; NiCl₂(PBu₃)₂ et un rapport molaire Ni/EADC égal à 1 : 20 on obtient, à une pression d'éthylène de 0,1 MPa et en présence d'ester méthylique de tournesol contenant 61,6% de linoléate de méthyle non conjugué, les résultats suivants

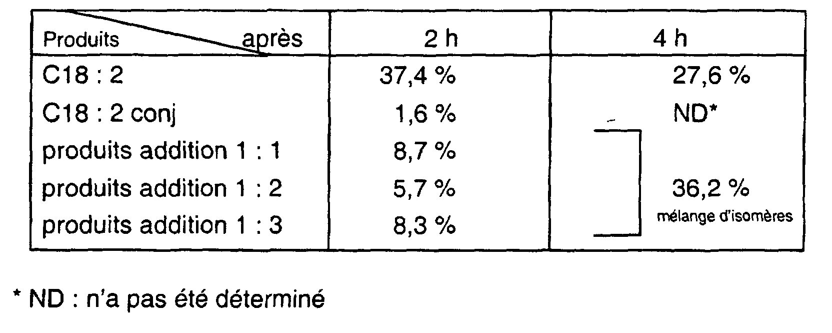

### EXEMPLE 11 :

On peut fabriquer le triéthylstéarate de triméthylolpropane (TMP) en faisant réagir l'éthyl-stéarate de méthyle avec du TMP.

Les propriétés physiques obtenues sont comparées avec différents esters de TMP obtenus à partir d'acides gras ramifiés, non ramifiés, saturés et polyinsaturés

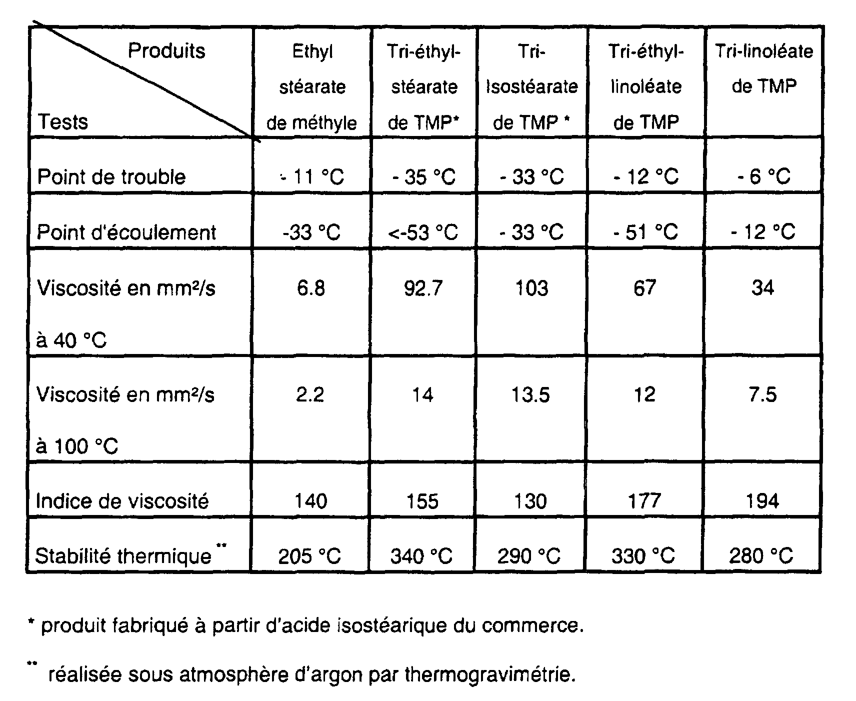

On remarque que le tri-éthyl stéarate de TMP (produit purifié par hydrogénation et cristallisation) possède des propriétés beaucoup plus intéressantes que le produit non purifié (tri-éthyl linoléate de TMP). De plus, la conservation des insaturations de la chaîne grasse est un handicap pour la stabilité à l'oxydation de la molécule.

### EXEMPLE 12 :

Les sels de sodium et les monoglycérides des acides ramifiés possèdent des tensions interfaciales très basses par rapport à l'eau et au gazole.

Les résultats sont consignés dans le tableau suivant.

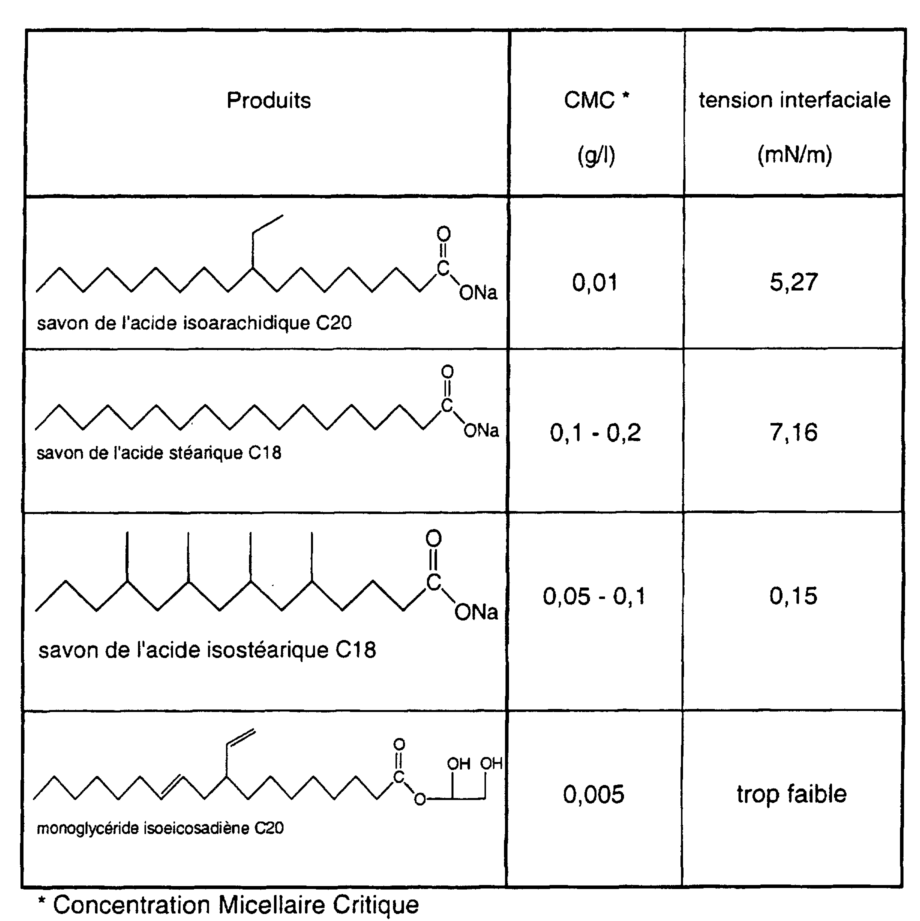

Quelques milligrammes de monoglycéride de l'acide ramifié peuvent réduire presque totalement la tension interfaciale entre l'eau et le gazole.

## Revendications

1. Procédé d'obtention d'un codimère **caractérisé en ce que** l'on additionne au moins un composé monooléfinique sur un corps gras comportant au moins deux liaisons éthyléniques conjuguées ou non conjuguées, en présence d'un système catalytique comprenant :
• au moins un composé du nickel choisi parmi les sels inorganiques ou organiques, bivalents, monovalents ou zérovalents du nickel ;
• au moins un composé réducteur choisi parmi les composés organoaluminiques, les composés organomagnésiens et les aluminoxanes ;
• et/ou au moins un acide de Lewis ;
• et au moins un ligand choisi parmi :
- les composés du phosphore de formule PRₘX₃₋ₘ, avec m = 2 ou 3 ; R = aryle ou alkyle ; X = halogène, les phosphites de formule P(OR)₃, avec R = aryle ou alkyle, les oxydes de phosphine POR₃, les diphosphines de formule R₂P-(CH₂)ₙ-PR₂, avec R = aryle ou alkyle ; n = 0 - 4 ;
- les composés analogues de l'arsenic et de l'antimoine ; et
- les ligands azotés, amidiques, les imines ou diminues et les dérivés pyridiniques.

2. Procédé selon la revendication 1 **caractérisé en ce que** ledit composé monoléfinique est un hydrocarbure monooléfinique choisi parmi l'éthylène, le propylène et le butène-1.

3. Procédé selon la revendication 1 et 2 **caractérisé en ce que** ledit corps gras comportant au moins deux liaisons éthyléniques est choisi parmi les corps gras diéniques ou polyéniques conjugués ou conjugables, le nombre d'atomes de carbone de la chaîne grasse comportant de 18 à 26 atomes de carbone sur la chaîne qui porte le groupe carboxylique, celui-ci étant lié à un alcool mono-, di-, tri- ou tétrafonctionnel de 1 à 22 atomes de carbone.

4. Procédé selon la revendication 3 **caractérisé en ce que** l'alcool est choisi parmi le méthanol, l'éthanol le néopentylglycol, le triméthylolpropane, le 2-éthylhexanol, le glycérol et les alcools gras de C₁₆ à C₂₂.

5. Procédé selon l'une des revendications de 1 à 4 **caractérisé en ce que** le système catalytique comporte, à titre de composé de nickel, un halogénure, un acétylacétonate ou un carboxylate et, à titre de réducteur, un système à base d'alkylaluminium, substitué ou non, un aluminoxane ou un hydrure d'aluminium, le rapport molaire entre le nickel et le réducteur étant de 1 à 30, de préférence de 4 à 15.

6. Procédé selon l'une des revendications de 1 à 5 **caractérisé en ce que** le ligand introduit a pour formule :
PR_{3,} P(OR)₃ ou R₂P-(CH₂)ₙ-PR₂,
avec n = 1, 2, 3 ou 4 et R = alkyle ou aryle, le rapport molaire entre le nickel et le ligand étant de 1 à 10, de préférence de 1 à 4.

7. Procédé selon l'une des revendications de 1 à 6 **caractérisé en ce que** l'ester engagé est présent sous forme conjuguée, ou est conjugué au cours de l'addition avec le même système que celui qui permet l'addition d'oléfine.

8. Procédé selon l'une des revendications 1 à 7 **caractérisé par le fait que** le catalyseur est un système mixte obtenu par réaction d'une part du complexe Ni(dioléfine)₂, où dioléfine = cyclooctadiène, norbornadiène, ou tout autre diène, et d'autre part d'un acide de Bronstedt ou de Lewis du type : HCl, HAlCl₄, HAlF₄, HPF₆, HSbF₆ et d'un ligand phosphinique tel que défini dans la revendication 6.

9. Procédé selon l'une des revendications 1 à 8 **caractérisé par le fait que** le composé oléfinique produit par codimérisation contient un chaînon ou plusieurs chaînons et dont un des chaînons peut avoir une longueur double ou triple de celle de l'oléfine utilisée dans la codimérisation.

10. Procédé de préparation de composés ramifiés saturés par hydrogénation de composés ramifiés obtenus selon l'une des revendications 1 à 9, **caractérisé par le fait que** les composés mono-, di- et/ou tri-ramifiés insaturés sont hydrogénés totalement ou partiellement en présence du catalyseur de codimérisation ou d'un catalyseur connu d'hydrogénation, éventuellement après filtration ou élimination du catalyseur de codimérisation.

11. Procédé selon la revendication 10 **caractérisé par le fait que** les composés ramifiés saturés obtenus par hydrogénation sont ensuite purifiés après séparation des composés ramifiés par distillation et/ou par élimination des composés saturés non ramifiés par cristallisation dans un solvant.

## Patentansprüche

1. Verfahren zur Herstellung eines Codimers, **dadurch gekennzeichnet, dass** man mindestens eine Monoolefin-Verbindung an einen Fettkörper addiert, der mindestens zwei konjugierte oder nicht-konjugierte Ethylen-Bindungen aufweist, in Gegenwart eines katalytischen Systems, das umfasst:
• mindestens eine Nickel-Verbindung, ausgewählt aus bivalenten, monovalenten oder nullvalenten anorganischen oder organischen Salzen von Nickel;
• mindestens eine reduzierende Verbindung, ausgewählt aus aluminiumorganischen Verbindungen, magnesiumorganischen Verbindungen und Aluminoxanen;
• und/oder mindestens eine Lewis-Säure;
• und mindestens einen Liganden, ausgewählt aus
- Phosphor-Verbindungen der Formel PRₘX₃₋ₘ, worin m = 2 oder 3; R = Aryl oder Alkyl; X = Halogen; Phosphiten der Formel P(OR)₃, worin R = Aryl oder Alkyl; Phosphinoxiden POR₃, Diphosphinoxiden der Formel R₂P-(CH₂)ₙ-PR₂, worin R = Aryl oder Alkyl und n = 0 bis 4;
- analogen Verbindungen von Arsen und Antimon; und
- stickstoffhaltigen Amid-, Imin- oder Diimin-Liganden oder Pyridin-Derivaten.

2. Verfahren nach Anspruch 1, **dadurch gekennzeichnet, dass** die Monoolefin-Verbindung ein Monoolefin-Kohlenwasserstoff, ausgewählt aus Ethylen, Propylen und Buten-1 ist.

3. Verfahren nach den Ansprüchen 1 und 2, **dadurch gekennzeichnet, dass** der Fettkörper, der mindestens zwei Ethylen-Bindungen aufweist, ausgewählt wird aus konjugierten oder konjugierbaren Dien- oder Polyen-Fettkörpern, in denen die Anzahl der Kohlenstoffatome der Fettkette 18 bis 26 Kohlenstoffatome beträgt in der Kette, welche die Carboxylgruppe trägt, die an einen mono-, di-, tri oder tetrafunktionellen Alkohol mit 1 bis 22 Kohlenstoffatomen gebunden ist.

4. Verfahren nach Anspruch 3, **dadurch gekennzeichnet, dass** der Alkohol ausgewählt wird aus der Gruppe Methanol, Ethanol, Neopentylglycol, Trimethylolpropan, 2-Ethylhexanol, Glycerin und der C₁₆-C₂₂-Fettalkohole.

5. Verfahren nach einem der Ansprüche 1 bis 4, **dadurch gekennzeichnet, dass** das katalytische System als Nickel-Verbindung ein Halogenid, ein Acetylacetonat oder ein Carboxylat und als Reduktionsmittel ein System auf Basis von substituiertem oder unsubstituiertem Alkylaluminium, ein Aluminoxan oder ein Aluminiumhydrid enthält, wobei das Molverhältnis zwischen dem Nickel und dem Reduktionsmittel 1 bis 30, vorzugsweise 4 bis 15, beträgt.

6. Verfahren nach einem der Ansprüche 1 bis 5, **dadurch gekennzeichnet, dass** der eingeführte Ligand die Formel hat:
PR₃, P(OR)₃ oder R₂P-(CH₂)ₙ-PR₂
worin n = 1, 2, 3 oder 4 und R = Alkyl oder Aryl, wobei das Molverhältnis zwischen Nickel und dem Liganden 1 bis 10, vorzugsweise 1 bis 4, beträgt.

7. Verfahren nach einem der Ansprüche 1 bis 6, **dadurch gekennzeichnet, dass** der gebundene Ester in konjugierter Form vorliegt oder im Verlaufe der Addition mit dem gleichen System wie dasjenige, das die Addition von Olefin erlaubt, konjugiert wird.

8. Verfahren nach einem der Ansprüche 1 bis 7, **dadurch gekennzeichnet, dass** der Katalysator ein gemischtes System ist, das hergestellt wurde durch Umsetzung einerseits des Komplexes Ni(diolefin)₂, worin das Diolefin für Cyclooctadien, Norbornadien oder irgendein anderes Dien steht, mit andererseits einer Bronstedt-Säure oder einer Lewis-Säure vom Typ HCl, HAlCl₄, HAlF₄, HPF₆, HSbF₆ und einem Phosphin-Liganden, wie er in Anspruch 6 definiert ist.

9. Verfahren nach einem der Ansprüche 1 bis 8, **dadurch gekennzeichnet, dass** die durch Codimerisation gebildete Olefin-Verbindung ein oder mehrere Kettenglieder enthält, wobei eines der Kettenglieder eine Länge haben kann, die das Doppelte oder Dreifache derjenigen des bei der Codimerisation verwendeten Olefins beträgt.

10. Verfahren zur Herstellung von gesättigten verzweigten Verbindungen durch Hydrierung von verzweigten Verbindungen, die nach einem der Ansprüche 1 bis 9 hergestellt worden sind, **dadurch gekennzeichnet, dass** ungesättigte einfach-, zweifach- und/oder dreifach verzweigte Verbindungen vollständig oder teilweise hydriert werden in Gegenwart eines Codimerisations-Katalysators oder eines bekannten Hydrierungs-Katalysators, gegebenenfalls nach dem Abfiltrieren oder Eliminieren des Codimerisations-Katalysators.

11. Verfahren nach Anspruch 10, **dadurch gekennzeichnet, dass** die durch Hydrierung erhaltenen gesättigten verzweigten Verbindungen anschließend gereinigt werden nach der Abtrennung der verzweigten Verbindungen durch Destillation und/oder durch Eliminierung der gesättigten nicht-verzweigten Verbindungen durch Kristallisation in einem Lösungsmittel.

## Claims

1. A process for obtaining a codimer, **characterised in that** at least one monoolefinic compound is added to a fatty substance that comprises at least two conjugated or unconjugated ethylene bonds, in the presence of a catalytic system that comprises
-- at least one nickel compound selected from the bivalent, monovalent or zerovalent, inorganic or organic nickel salts;
-- at least one reducing compound selected from the organoaluminium compounds, the organomagnesium compounds, and aluminoxanes;
-- and/or at least one Lewis acid;
-- and at least one ligand selected from:
the phosphorus compounds of formula PRₘX₃₋ₘ with m = 2 or 3; R = aryl or alkyl; X = halogen; phosphites of formula P(OR)₃, with R = aryl or alkyl; phosphine oxides POR₃, and diphosphines of formula R₂P-(CH₂)ₙ-PR₂, with R = aryl or alkyl; n = 0-4;
the analogous compounds of arsenic and antimony; and
the nitrogenous ligands, amide ligands, imines or diimines and the pyridine derivatives.

2. A process according to claim 1, **characterised in that** said monoolefinic compound is a monoolefinic hydrocarbon selected from ethylene, propylene, and 1-butene.

3. A process according to claims 1 and 2, **characterised in that** said fatty substance that comprises at least two ethylene bonds is selected from the diene or polyene fatty substances that are conjugated or can be conjugated, whereby the number of carbon atoms of the fatty chain comprises 18 to 26 carbon atoms on the chain that carries the carboxylic group, with the latter being linked to a mono-, di-, tri- or tetrafunctional alcohol with 1 to 22 carbon atoms.

4. A process according to claim 3, **characterised in that** the alcohol is selected from among methanol, ethanol, neopentylglycol, trimethylolpropane, 2-ethylhexanol, glycerol and the fatty alcohols of C₁₆ to C₂₂.

5. A process according to one of claims 1 to 4, **characterised in that** the catalytic system comprises, as the nickel compound, a halide, an acetylacetonate or a carboxylate and, as the reducing agent, a system with an alkylaluminium base, which may or may not be substituted, an aluminoxane or an aluminium hydrides, whereby the molar ratio between the nickel and the reducing agent is 1 to 30, preferably 4 to 15.

6. A process according to one of claims 1 to 5, **characterised in that** the ligand that is introduced has as its formula PR₃, P(OR)₃ or R₂P-(CH₂)ₙ-PR₂, with n = 1, 2, 3 or 4 and R = alkyl or aryl, whereby the molar ratio between the nickel and the ligand is 1 to 10, preferably 1 to 4.

7. A process according to one of claims 1 to 6, **characterised in that** the ester that is employed is present in conjugated form or is conjugated during addition with the same system as the one that allows the addition of olefin.

8. A process according to one of claims 1 to 7, **characterised in that** the catalyst is a mixed system that is obtained by reaction, on the one hand, of the complex Ni(diolefin)₂, where diolefin = cyclooctadiene, norbornadiene, or any other diene, and, on the other hand, a Bronsted or Lewis acid such as HCl, HAlCl₄, HAlF₄, HPF₆, HSbF₆ and a phosphine ligand as defined in claim 6.

9. A process according to one of claims 1 to 8, **characterised in that** the olefinic compound that is produced by codimerisation contains one or more links, of which one of the links can have a length that is double or triple that of the olefin that is used in the codimerisation.

10. A process for preparation of saturated branched compounds by hydrogenation of branched compounds that are obtained according to one of claims 1 to 9, **characterised in that** the unsaturated, mono-, di- and/or tri-branched compounds are hydrogenated totally or partially in the presence of the codimerisation catalyst or a known hydrogenation catalyst, optionally after filtration or elimination of the codimerisation catalyst.

11. A process according to claim 10, **characterised in that** the saturated branched compounds that are obtained by hydrogenation are then purified after the branched compounds are separated by distillation and/or by elimination of the unbranched saturated compounds by crystallization in a solvent.
